# FASCICULE DE BREVET EUROPEEN

(11) **EP 1 214 948 B1**
(45) Date de publication et mention de la délivrance du brevet: **29.09.2004**
(21) Numéro de dépôt: 01403156.1
(22) Date de dépôt: 06.12.2001
(51) Int. Cl.: A01N 31/08, A01N 65/00, A01N 25/20, A61L 9/02

(54) **Composition anti-bactérienne pour lutter contre les bactéries contenues dans l'air et procédé correspondant**
Antibakterielle Zusammensetzung zur Bekämpfung von in der Luft schwebenden Bakterien und entsprechendes Verfahren
Antibacterial composition for controlling airborne bacteria and corresponding procedure

(30) Priorité: 14.12.2000 FR 0016338
(43) Date de publication de la demande: 19.06.2002
(73) Titulaire: PRODUITS BERGER, 75116 Paris (FR)
(72) Inventeur: Lehoux, Jannick, 27370 Thuit-Signol (FR); Gomez, Corinne, 27400 Louviers (FR)
(74) Mandataire: Rémont, Claude

(56) Documents cités:
- WO-A-98/37922
- FR-A- 2 179 664
- FR-A- 2 540 382
- FR-A- 2 598 615
- FR-A- 2 779 509
- GB-A- 2 181 351
- US-A- 4 983 635
- DATABASE WPI Section Ch, Week 199715 Derwent Publications Ltd., London, GB; Class C03, AN 1997-161358 XP002178693 & JP 09 030906 A (KING KAGAKU KK), 4 février 1997 (1997-02-04)

## Description

La présente invention concerne une composition destinée à être diffusée pour lutter contre les bactéries contenues dans l'air d'une enceinte à traiter. L'invention concerne également un procédé de lutte contre les bactéries comprenant une étape de diffusion de ladite composition dans une enceinte. Elle se rapporte enfin à une utilisation d'un flacon à combustion catalytique pour la mise en oeuvre d'un tel procédé.

Dans la présente invention, la lutte contre les bactéries couvre l'activité bactéricide, qui se dit d'une substance qui a la propriété de tuer les bactéries, ainsi que l'activité bactériostatique qui se dit d'une substance qui freine le développement des dites bactéries. Ces deux activités seront visées par le vocable "anti-bactérien,".

Les techniques connues à ce jour pour lutter contre les bactéries mettent en oeuvre des produits désinfectants appliqués, en général, directement sur les surfaces contaminées.

Parmi le large éventail de composés connus pour avoir une activité conservatrice et anti-bactérienne, on peut citer le 2-phénylphénol, connu commercialement sous le nom de "préventol O extra" ou encore E 231. Ce composé entre dans la fabrication de nombreux désinfectants et détergents mais également convient à la conservation d'un grand nombre de produits. Le 2-phénylphénol possède un vaste spectre d'efficacité contre les bactéries, levures et autres moisissures.

L'essence de thym rouge extra, dans sa forme commerciale, est un autre composé connu pour avoir une activité anti-bactérienne. Elle est employée dans certains produits pharmaceutiques tels que les dentifrices, les antiseptiques pour les bains de bouche,... L'essence de thym rouge extra contient entre 40 et au maximum 52% en poids de thymol naturel, 20% environ de paracymène, 3 à 4% environ de carvacrol, le reste étant constitué de terpènes et d'alcools.

On connaît également le thymol de synthèse.

Ces deux composés sont donc bien connus comme anti-bactériens et agissent par contact au niveau des surfaces à traiter.

Le document JP-09 030609 décrit une composition avec effet antibactérien pour la répulsion des cafards, comprenant de la 2-undécanone à titre d'agent répulsif et de l'ortho-phénylphénol et/ou du thymol à titre d'agent antibactérien. Cette composition est imprégnée dans une support qui est ensuite disposé à l'endroit où l'effet répulsif est désiré pour agir par contact à l'encontre des parasites considérés.

Des dispositifs existent également pour assainir l'air. Ils se présentent sous la forme d'ioniseurs, de purificateurs, de bombes aérosols ou de pulvérisateurs.

Les ioniseurs sont plus spécifiquement adaptés pour lutter contre la poussière. L'inconvénient est que leur utilisation contribue à l'émission d'ozone pouvant représenter un danger pour l'homme.

Les purificateurs d'air agissent plus spécifiquement sur les poussières de l'air.

Les bombes aérosols et pulvérisateurs, quant à eux, permettent de diffuser, dans l'air et sous forme de gouttelettes plus ou moins fines, les produits qu'ils contiennent, qui agissent également principalement par contact, compte tenu de la dimension des gouttelettes.

Ces gouttelettes, de grosse taille, offrent une surface spécifique d'action inférieure à celle que l'on pourrait obtenir avec une plus grande quantité de plus petites gouttelettes.

Rien n'interdit cependant d'envisager la diffusion de compositions anti-bactériennes au moyen de bombes aérosols ou de pulvérisateurs. Toutefois, l'éventuel effet anti-bactérien de tels dispositifs contre les bactéries contenues dans l'air d'une enceinte, n'a été ni constaté ni démontré à ce jour. Par ailleurs, ce mode de diffusion reste très local et ne peut être que curatif. De plus, les concentrations en substances actives restent assez élevées et sont donc relativement nocives et/ou gênantes pour l'utilisateur.

La présente invention vise à pallier l'ensemble des inconvénients mentionnés ci-dessus et propose une composition destinée à être diffusée dans l'air d'une enceinte à traiter pour lutter contre les bactéries contenues dans l'air de cette enceinte.

Suivant l'invention, cette composition comprend du 2-phénylphénol, du thymol, naturel et/ou de synthèse, et un solvant adapté à être diffusé par vaporisation à chaud, le 2-phénylphénol et le thymol étant dissous dans ledit solvant qui peut comprendre, par exemple, de l'alcool isopropylique.

Il a été constaté de façon surprenante l'existence d'une synergie entre ces deux composés permettant d'obtenir une action anti-bactérienne sur les germes de l'air nettement supérieure à l'action anti-bactérienne conférée par la diffusion par vaporisation à chaud dans l'air du seul 2-phénylphénol ou du seul thymol.

Généralement, la composition comprend une proportion pondérale comprise entre 0,05 et 1% environ de 2-phénylphénol et une proportion pondérale comprise entre 0,05 et 5% environ de thymol, le reste comprenant le solvant.

Des teneurs en 2-phénylphénol et thymol qui seraient inférieures aux valeurs minimales indiquées ci-dessus ne permettent probablement pas d'atteindre l'objectif ambitieux recherché, fixé de façon arbitraire à une réduction d'au moins environ 75%, de préférence d'au moins 80% des bactéries contenues dans l'air d'une enceinte à traiter et ce, 24 heures après la diffusion, pendant un temps relativement court, de l'ordre d'une vingtaine de minutes, d'une composition anti-bactérienne selon l'invention.

Des teneurs en 2-phénylphénol et thymol supérieures aux valeurs maximales indiquées ci-dessus permettent bien évidemment d'obtenir l'effet antibactérien recherché et il est donc tout à fait envisageable de diffuser des compositions anti-bactériennes selon l'invention comprenant des proportions pondérales de ces deux composés supérieures, voire nettement supérieures.

Cependant, ces valeurs maximales ont été déterminées pour tenir compte de différents paramètres, notamment économiques, afin que le coût en composés actifs ne soit pas rédhibitoire sur le plan commercial, mais également pour des raisons plus pratiques.

En particulier, dans le cas où l'on envisage la diffusion, par combustion catalytique, de compositions anti-bactériennes selon l'invention, il est indispensable d'éviter un vieillissement prématuré du brûleur catalytique utilisé.

La diffusion des compositions anti-bactériennes selon l'invention doit, dans la mesure du possible, éviter de générer des fumées ou des composés qui présentent une toxicité connue pour l'utilisateur ; accessoirement l'odeur générée doit plaire à l'utilisateur.

D'autres critères peuvent également être pris en compte pour limiter le pourcentage pondéral de l'un et/ou de l'autre des composés anti-bactériens employés dans le cadre de la présente invention, comme la solubilité ou l'aspect de la composition.

Dans une version avantageuse, la composition comprend une proportion pondérale comprise entre 0,1 et 0,3% environ de 2-phénylphénol, et une proportion pondérale comprise entre 0,2 et 2% environ de thymol.

Dans une version préférée, la composition comprend une proportion pondérale égale à 0,2% environ de 2-phénylphénol, et une proportion pondérale comprise entre 0,5 et 1% environ de thymol.

Les quantités de 2-phénylphénol et de thymol sont dans tous les cas très inférieures à celles des produits anti-bactériens couramment employés avec les procédés de diffusion connus.

Le procédé selon l'invention engendre beaucoup moins d'effets secondaires pour l'utilisateur et présente une toxicité très inférieure à celle des procédés connus.

Selon un mode préféré de l'invention, la composition comprend une proportion pondérale totale de 2-phénylphénol et de thymol comprise entre 0,1 et 5% environ, de préférence entre 0,2 et 1,2% environ.

La quantité totale de produits actifs employée dans le cadre de la présente invention est bien inférieure à l'art antérieur, et peut atteindre une réelle efficacité pour des concentrations pondérales dix fois moindres.

La présente invention concerne également un procédé de lutte contre les bactéries contenues dans l'air d'une enceinte à traiter. Ce procédé comprend une étape de diffusion, dans l'air de cette enceinte, d'une composition anti-bactérienne.

Selon l'invention, on diffuse la composition comprenant du 2-phénylphénol, de thymol et un solvant par vaporisation à chaud, le 2-phénylphénol et le thymol étant dissous dans ledit solvant, de manière telle que ledit solvant se trouve entièrement à l'état gazeux.

Avantageusement, on diffuse ladite composition au moyen d'un brûleur à combustion catalytique, le solvant étant combustible et comprenant, par exemple, de l'alcool isopropylique.

L'invention concerne également l'utilisation, pour la mise en oeuvre du procédé de lutte contre les bactéries selon l'invention, d'un flacon à combustion catalytique, adapté à contenir une composition combustible et à recevoir, sur une embase fixée à son goulot, un brûleur à combustion catalytique, ledit brûleur comportant une cavité sensiblement axiale ouverte vers le bas et recevant une mèche trempant dans ladite composition, le brûleur pouvant présenter une gorge annulaire sensiblement axiale s'étendant depuis la surface supérieure du brûleur et séparant la zone périphérique annulaire supportant le catalyseur de la zone centrale sans catalyseur formant zone de vaporisation, la cavité étant ménagée dans une masse de matière céramique exempte de catalyseur, et/ou un canal pour mettre à l'atmosphère la partie supérieure de la cavité recevant la mèche.

D'autres avantages et particularités de l'invention résulteront de la description qui va suivre, donnée à titre d'exemples non limitatifs et faite en référence aux figures annexées dans lesquelles :
- la figure 1 est une vue schématique en élévation d'un flacon équipé d'un brûleur à combustion catalytique qui peut être utilisé pour la mise en oeuvre du procédé selon la présente invention ;
- la figure 2 est une vue schématique agrandie en coupe axiale du brûleur représenté à la figure 1, dans un mode préférentiel de mise en oeuvre du présent procédé de la présente invention ;
- la figure 3 est une vue semblable à la figure 2 représentant un mode de réalisation d'un support du brûleur de la figure 2 ;
- la figure 4 est une vue semblable à la figure 2 représentant un mode de réalisation d'une embase adaptée à recevoir le support de la figure 3 ;
- la figure 5 représente les courbes I à IV de mesures de l'efficacité destructrice en fonction du temps exprimé en heures lors des essais 1 à 4 ;
- la figure 6 représente les courbes II et V à VIII de mesures de l'efficacité destructrice en fonction du temps exprimé en heures lors des essais 2 et 5 à 8.

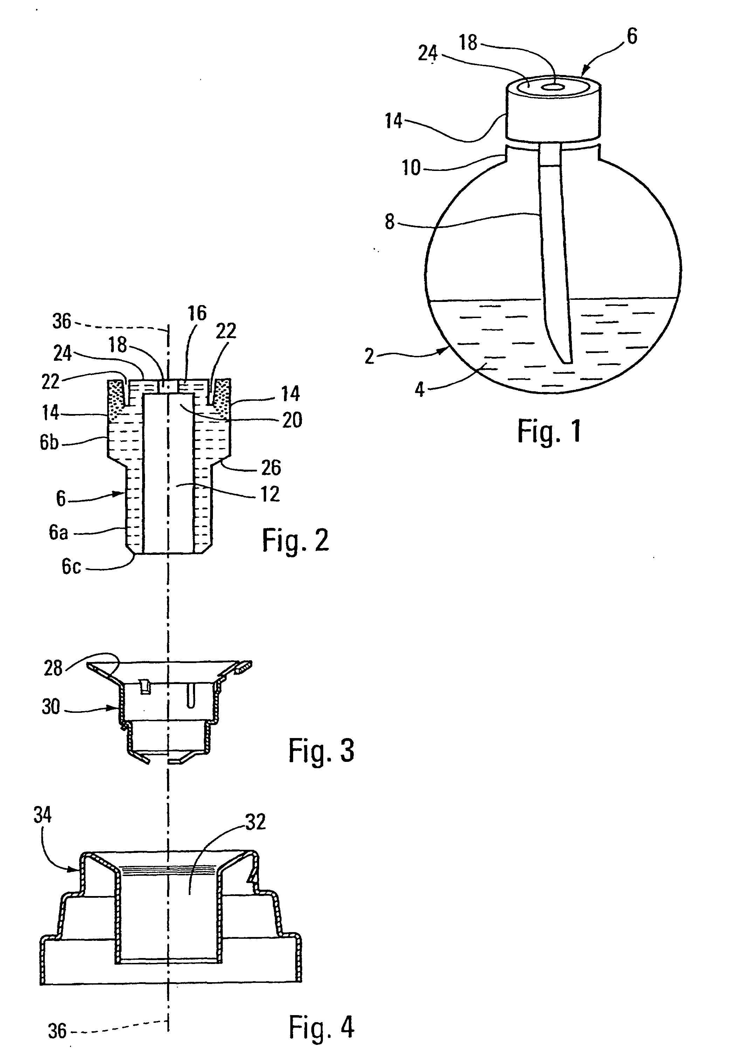

On a représenté à la figure 1 un flacon 2 à combustion catalytique au moyen duquel les essais qui vont être décrits ci-après ont été réalisés.

Ce flacon 2 à combustion catalytique est adapté à contenir une composition combustible 4 et à recevoir à sa partie supérieure un brûleur 6 à combustion catalytique recevant une mèche 8 trempant dans ladite composition 4.

Le flacon 2 peut être un flacon de forme quelconque présentant un goulot 10 sur lequel est adapté le brûleur 6.

La composition combustible 4 comprend un solvant qui appartient habituellement à la famille des alcools, et qui est par exemple de l'alcool isopropylique, mais peut également comporter tout autre combustible liquide approprié. De préférence, ce combustible liquide doit être tel que sa vaporisation et sa combustion catalytique ne dégagent pas de produits présentant une grande toxicité pour l'utilisateur dans les conditions normales de diffusion.

L'opération de diffusion est effectuée par vaporisation à chaud du solvant.

La composition combustible 4 selon l'invention peut éventuellement comprendre en outre un ou plusieurs autres composés, naturels ou de synthèse, par exemple des composés couramment diffusés au moyen d'un système à combustion catalytique et, en particulier, une matière parfumée.

La mèche 8 est une mèche connue quelconque, par exemple une mèche en coton. La mèche peut également être une mèche en matière minérale, par exemple une mèche en fibres minérales.

Comme représenté en détail à la figure 2, le brûleur 6 à combustion catalytique est un brûleur en matière céramique poreuse, par exemple à base de kaolin.

Le brûleur 6 comporte à sa partie inférieure 6a une cavité 12 sensiblement axiale adaptée à recevoir une mèche 8 destinée à amener au brûleur 6 la composition combustible 4. La cavité 12, qui débouche à l'extrémité inférieure 6c du brûleur 6, s'étend axialement sur une grande partie de la dimension axiale du brûleur 6.

Le brûleur 6 comporte à sa partie supérieure 6b une zone périphérique annulaire 14 supportant un catalyseur, par exemple à base d'un métal appartenant au groupe VIII du tableau de la classification périodique des éléments. La zone périphérique annulaire 14 entoure une zone centrale 16 sans catalyseur formant zone de vaporisation.

C'est au niveau de cette zone de vaporisation 16, qui est chauffée par conduction par la zone périphérique 14 dans laquelle s'effectue la combustion catalytique, que la composition 4 est diffusée dans l'air de l'enceinte.

La composition combustible 4 traverse la mèche 8 ainsi que les pores du brûleur par capillarité.

On admet que la composition combustible 4 qui traverse la zone périphérique 14, où se produit la combustion catalytique, est essentiellement brûlée et dénaturée.

Dans le cas présent mettant en oeuvre un flacon 2 à combustion catalytique, la diffusion par vaporisation s'entend donc de la vaporisation de la composition combustible 4 qui traverse la zone de vaporisation 16.

Dans l'exemple représenté à la figure 2, le brûleur 6 comporte dans sa partie supérieure 6b, au moins un canal 18 ouvert, faisant communiquer la partie supérieure 20 de la cavité 12 avec l'atmosphère.

Dans cet exemple et de façon classique, le brûleur 6 présente une gorge annulaire 22 sensiblement axiale s'étendant depuis la surface supérieure 24 du brûleur 6 vers le bas suivant un diamètre supérieur à celui de la cavité 12.

On sait que cette gorge annulaire 22 sépare nettement la zone périphérique annulaire 14, qui supporte le catalyseur, de la zone centrale 16 de la partie supérieure 6b.

Le brûleur 6 présente un épaulement périphérique 26 reçu par un épaulement complémentaire 28 d'un support 30 représenté à la figure 3.

Le support 30 peut être introduit directement à l'intérieur du goulot 10 du flacon 2.

Habituellement, le support 30 est introduit dans le trou central 32 d'une embase 34, comme schématisée à la figure 4, cette embase 34 étant adaptée à être fixée au goulot 10 du flacon 2.

Le support 30 a pour principal effet d'inciter l'utilisateur du brûleur 6 à mettre en place parfaitement le support 30 et le brûleur 6 sur l'embase 34 selon l'axe commun 36 au goulot 10, à l'embase 34, au support 30 et au brûleur 6.

Rien n'interdit d'envisager d'autres structures de flacon associé à un brûleur catalytique ; en particulier, les variantes de flacons décrites dans la demande internationale PCT/FR 99/00937 peuvent être avantageusement mises en oeuvre dans le cadre de la présente invention.

Différents essais ont été réalisés et ont permis de démontrer l'activité anti-bactérienne intense, contre les bactéries contenues dans l'air d'une enceinte à traiter, d'une composition comprenant du 2-phénylphénol et du thymol dissous dans un solvant, et de mettre en évidence la réelle synergie procurée par la diffusion, dans l'air de cette enceinte, de manière telle que le solvant se trouve entièrement à l'état gazeux, d'une telle composition dans la lutte contre les bactéries contenues dans cet air.

De façon générale, l'activité anti-bactérienne d'une composition est déterminée par la mesure de l'efficacité destructrice (ED) définie par la formule ED=log₁₀ (N_{o/}N), dans laquelle Nₒ est le nombre initial de bactéries relevé préalablement à la diffusion de la composition considérée, et N le nombre de bactéries relevé à un instant t, compté à partir de l'initialisation de ladite diffusion.

Bien que pouvant atteindre dans certains cas des valeurs de l'ordre de 0,7, l'efficacité destructrice obtenue au cours des toutes premières heures qui suivent la diffusion de chaque composition testée, ne présente guère d'intérêt dans le cas où elle diminue par la suite très rapidement par recontamination. En effet, un tel résultat correspond à celui que l'on peut obtenir avec les traitements par contact connus et décrits précédemment.

En l'absence de tout appareil de mesure normalisé permettant une mesure fiable, les essais 1 à 8 ont été réalisés conformément au mode opératoire suivant : la composition à tester a été introduite dans un diffuseur placé sur une table, elle-même disposée au milieu d'une salle de 50 m³.

Les prélèvements d'air, en vue des mesures de population bactérienne, sont effectués au moyen d'un aérobiocollecteur disposé à 2,30 m du flacon, légèrement en contrebas par rapport audit flacon.

Un aérobiocollecteur est un dispositif aspirant l'air à raison de 100 l/min dans lequel se trouve un piège tel qu'une boîte de Piétri contenant de la gélose, permettant de récupérer les bactéries contenues dans l'air.

La population bactérienne initiale est relevée pour chacun des essais préalablement à la mise en oeuvre de la combustion catalytique.

Lors des essais 1 à 7, la composition testée a été introduite dans un flacon du type de celui représenté à la figure 1, ce flacon étant muni d'un brûleur à combustion catalytique tel que décrit à la figure 2.

La combustion catalytique est mise en oeuvre de façon continue pendant 25 minutes puis interrompue. La population bactérienne est mesurée à intervalles de temps réguliers, comptés à partir de l'initialisation de la combustion catalytique, puis l'ED correspondante déterminée.

Il est précisé que le solvant utilisé dans toutes les compositions des essais 1 à 7 est l'alcool isopropylique à 90% en volume dans l'eau.

Il est connu depuis longtemps de diffuser, par combustion catalytique, un produit anti-bactérien. Cependant, les essais 2 à 4 vont permettre de quantifier plus précisément cette activité anti-bactérienne.

Au préalable, un premier essai, ou essai 1, a été conduit pour déterminer l'activité anti-bactérienne du seul solvant.

L'essai 1 a été réalisé en n'introduisant que de l'alcool isopropylique dans le flacon, conformément au mode opératoire ci-dessus.

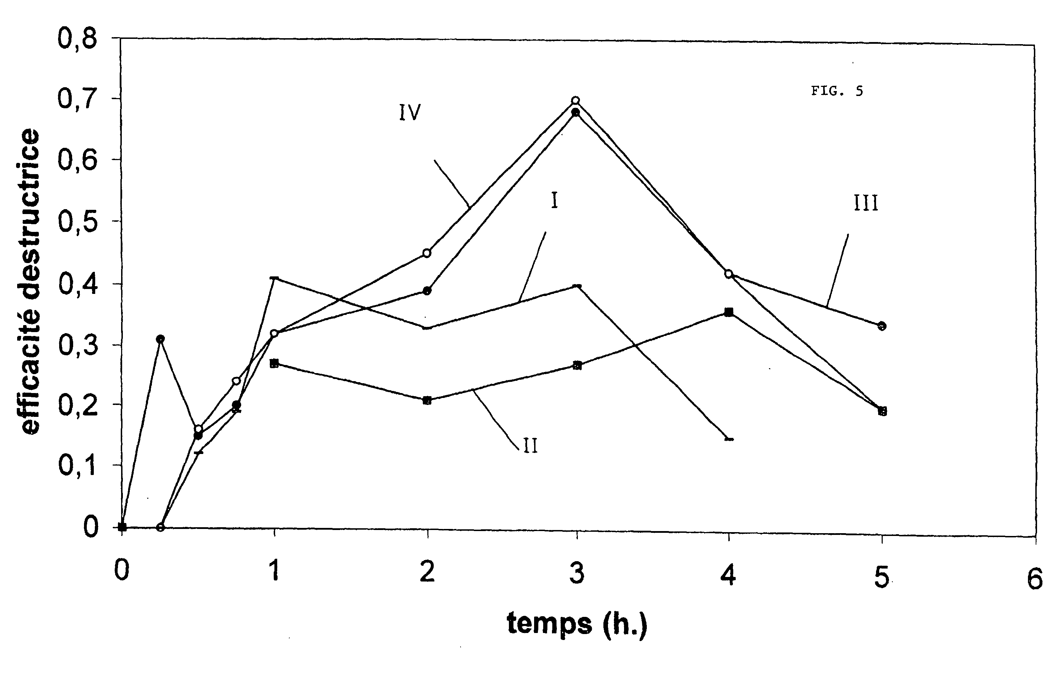

Les mesures relevées lors de ce premier essai sont représentées par la courbe I de la figure 5.

Cette courbe I indique une efficacité destructrice ED faible, nettement en dessous des valeurs recherchées d'au moins 0,6.

En effet, la valeur D'ED maximale, atteinte au bout d'une heure, est de l'ordre de 0,41. Ceci indique qu'au plus 60% des germes banaux de l'air, tels que *Rhodotorula rubra* (levures), *Aspergillus niger, Penicillium* (moisissures), sont détruits une heure après l'allumage du brûleur à combustion catalytique.

Une faible activité anti-bactérienne perdure pendant environ 3 à 4 heures après cet allumage puis on constate que l'air se recontamine progressivement. L'ED devient nulle au bout de 5 heures, ce qui signifie que l'air au voisinage du diffuseur a été progressivement recontaminé pour atteindre la population bactérienne initiale (Nₒ).

Les résultats de cet essai 1 indiquent que l'alcool isopropylique a bien, sur la contamination microbienne de l'air, un certain effet probablement localisé au voisinage du diffuseur et de durée très faible.

L'objectif minimum à atteindre, tel que recherché, est une destruction, après au minimum 24 heures comptées à partir de l'initialisation de la diffusion de la composition anti-bactérienne, d'au moins 3/4 des bactéries initialement présentes, ce qui correspond à une valeur d'ED minimale recherchée de l'ordre de 0,60, valeur qui n'est jamais atteinte dans l'essai 1.

L'ED mesurée dans le cadre de l'essai 2, pour une composition contenant 1% en poids d'essence de thym rouge, c'est-à-dire qui comporte au maximum 0,52% en poids de thymol naturel, est relativement faible.

Comme l'indique la courbe II de la figure 5 qui représente l'évolution des valeurs de l'efficacité destructrice relevées en fonction du temps lors de cet essai 2, l'ED maximale atteinte, 23 heures après l'initialisation de la combustion catalytique, est de 0,52. 55 heures après cette même initialisation, l'ED devient nulle.

La comparaison des valeurs d'ED obtenues dans le cadre des essais 1 et 2 montre que la présence d'essence de thym rouge dans l'alcool isopropylique améliore très légèrement l'activité anti-bactérienne de la composition : on atteint une destruction maximale de 70% des bactéries contenues dans l'air lors de ce second essai, contre seulement 60% dans l'essai 1.

Toutefois, les valeurs minimales recherchées dans le cadre de la présente invention ne sont pas atteintes.

On constate cependant que l'introduction d'essence de thym rouge dans l'alcool isopropylique prolonge l'activité anti-bactérienne de la composition dans le temps ; en effet, l'ED ne devient nulle qu'au bout de 55 heures et non plus seulement 5 heures après l'initialisation de la combustion catalytique comme dans le cas de l'essai 1.

Ceci confirme l'activité anti-bactérienne au demeurant connue, de l'essence de thym rouge.

Lors des essais 3 et 4, deux compositions comprenant respectivement 0,2 et 1% en poids de 2-phénylphénol ont été testées.

Les courbes d'ED en fonction du temps qui ont été obtenues, respectivement référencées III (essai 3) et IV (essai 4) sur la figure 5, sont tout à fait comparables.

Le fait de quintupler la proportion de 2-phénylphénol dans la composition ne permet pas d'observer une amélioration quantifiable de l'ED, tant du point de vue de l'activité anti-bactérienne immédiate (effet curatif) que de l'activité anti-bactérienne rémanente (effet préventif).

Par ailleurs, et bien que l'ED atteigne une valeur d'ED maximale de l'ordre de 0,7, permettant ainsi de réduire de 80% le nombre de bactéries, on constate que cet effet intervient très rapidement, 3 heures après l'initialisation de la combustion catalytique.

Aucun effet rémanent de l'activité anti-bactérienne n'est mis en évidence : dans les essais 3 et 4, la valeur de l'ED devient nulle, 8 heures après l'initialisation de la combustion catalytique.

En conséquence, l'activité anti-bactérienne de compositions contenant du 2-phénylphénol n'est absolument pas satisfaisante au regard des exigences requises pour un assainissement de l'air d'une enceinte

On constate donc que dans les essais 1 à 4, les compositions testées ne permettent pas de façon générale d'atteindre une réduction importante de la population bactérienne. Et bien qu'une valeur de 0,7 d'efficacité destructrice ait effectivement été mesurée lors des essais 3 et 4, l'activité anti-bactérienne de telles compositions ne s'inscrit pas dans la durée.

L'activité anti-bactérienne reste vraisemblablement localisée autour du flacon à combustion catalytique, si bien qu'il se produit une recontamination de l'air de l'enceinte, dès l'arrêt de ladite combustion. Cette recontamination est par ailleurs d'autant plus rapide que peu de bactéries ont été détruites.

L'insuffisance de l'activité anti-bactérienne des compositions des essais 1 à 4, pour un usage spécifique anti-bactérien, est corroborée par des mesures complémentaires conduites sur des bactéries pathogènes utilisées dans les normes AFNOR, de telles mesures ayant abouti à une ED nulle.

Les essais 1 à 4 illustrent ainsi ce que l'on peut considérer comme constituant l'état de la technique à la date de la présente invention.

L'objectif recherché par la présente invention a été fixé arbitrairement à une réduction d'au moins 75% des bactéries initialement présentes dans l'air de l'enceinte à traiter, la valeur de l'efficacité destructrice ED à atteindre, après 24 heures comptées à partir de l'initialisation de la combustion catalytique, est d'au moins 0,6, et de préférence supérieure à 0,7.

Cet objectif est effectivement atteint dans les conditions des essais 5 à 8.

Lors de l'essai 5, une composition selon l'invention comprenant à la fois du 2-phénylphénol et de l'essence de thym rouge, dans des proportions pondérales respectives de 0,2 et 1% (ou au maximum 0,5% en thymol naturel) a été testée.

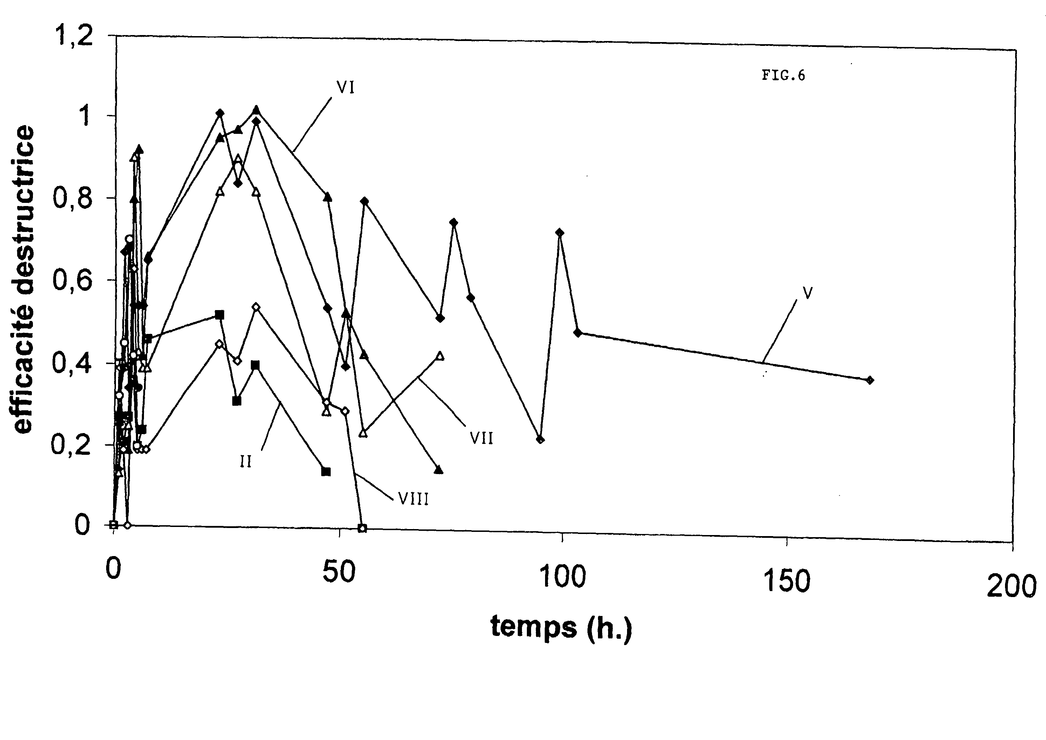

La courbe V représentée à la figure 6 montre que l'ED mesurée lors de l'essai 5 atteint une valeur maximale de 1,01, ce qui signifie que plus de 90% des bactéries ont été détruites, et ce 23 heures après l'initialisation de la combustion catalytique.

L'essai 5 fait nettement apparaître une synergie surprenante et inattendue entre l'essence de thym rouge et le 2-phénylphénol, synergie que les résultats des précédents essais 2, 3 et 4 ne permettaient pas de présumer.

En effet, 23 heures après l'initialisation de la combustion catalytique, l'ED atteignait au mieux la valeur de 0,52 dans l'essai 2, alors qu'elle était déjà égale à 0 dans les cas des essais 3 et 4.

Cet essai 5 met également en évidence un phénomène de rémanence surprenant et particulièrement intéressant. Bien plus, la valeur de l'ED de la composition de l'essai 5, mesurée à 55 heures est de 0,8, ce qui correspond encore à une réduction de la population bactérienne de 84%, bien au-delà de l'objectif de durée fixé arbitrairement à 24 heures.

168 heures après l'initialisation de la combustion catalytique, la valeur de l'ED de l'essai 5 est encore de 0,4, soit une réduction de 60%.

On peut supposer que cette rémanence est le résultat d'une action anti-bactérienne rapide et intense dans tout le volume de l'enceinte traitée.

L'essai 6 a été conduit avec une composition selon l'invention comprenant du 2-phénylphénol et du thymol de synthèse, dans des proportions pondérales respectives de 0,2 et 1%. Les mesures d'ED relevées au cours du temps sont représentées par la courbe VI sur la figure 6.

La valeur maximale d'ED atteinte à 31 heures est de 1,02 et correspond encore à une réduction de la population bactérienne de plus de 90%.

Les résultats présentés par cet essai 6 sont tout à fait satisfaisants au regard des exigences retenues par les inventeurs. Toutefois, le phénomène de rémanence est moins marqué que dans l'essai 5.

Lors de l'essai 7, la quantité de thymol de synthèse a été divisée par deux par rapport à celle employée lors de l'essai 6.

En se référant à la courbe VII sur la figure 6, on peut observer que les valeurs d'ED obtenues avec l'essai 7 sont comparables à celles obtenues dans l'essai 6, comme précédemment lors des essais 3 et 4, dans lesquels seule la proportion de thymol avait été modifiée.

La diminution de la quantité de thymol artificiel ne semble pas avoir de réelle influence au niveau de l'activité anti-bactérienne de la composition testée. Ainsi, une très faible proportion de thymol, combinée au 2-phénylphénol, permet d'obtenir l'activité anti-bactérienne recherchée et ce, dans des conditions économiques liées au coût de la matière intéressantes.

Par contre, la comparaison des essais 5 et 7, dans lesquels les proportions respectives de thymol naturel et de thymol artificiel sont de 0,5% en poids, montre un effet rémanent bien plus marqué, et donc une meilleure activité anti-bactérienne dans le temps, lorsque la composition est réalisée avec l'essence de thym rouge.

Ceci met donc en évidence une nouvelle synergie procurée par cette dernière composition selon l'invention, synergie probablement engendrée par un ou plusieurs des composés, autres que le thymol naturel, contenus dans l'essence de thym rouge.

On peut ainsi supposer que, parmi ces composés, le carvacrol, qui est un isomère du thymol et est par ailleurs connu comme anti-bactérien, participe à cette seconde synergie.

Dans le cadre de l'essai 8, un autre mode de diffusion a été mis en oeuvre ; dans cet essai, la vaporisation à chaud s'effectue par simple chauffage de la composition.

Le dispositif mis en oeuvre lors de cet essai 8 est conforme au diffuseur de parfum décrit dans la demande internationale PCT/FR 98/00391.

La composition anti-bactérienne testée, comprenant 0,2% en poids de 2-phénylphénol et 1% en poids d'essence de thym rouge dissous dans l'eau, a été introduite dans un récipient placé au même endroit de la salle que le dispositif employé lors des précédents essais 1 à 7.

Le récipient a ensuite été chauffé de manière à maintenir la composition à une température comprise entre 40 et 80°C, pendant une durée de 2 heures.

Une telle température permet d'assurer la vaporisation dans l'air de la composition, entraînée par le solvant, choisi pour le vaporiser aisément à une telle température.

Pour une diffusion par simple chauffage, il est également possible de positionner une source de chaleur sous un récipient contenant la composition selon l'invention, cette source de chaleur et le récipient étant adaptés à porter ladite composition à la température adéquate.

Les mesures de population bactérienne ont été effectuées avant, pendant, puis après la diffusion de la composition testée, selon les mêmes modalités de prélèvement que pour les essais 1 à 7.

Comme l'indique la courbe VIII portée sur la figure 6, la valeur maximale d'ED obtenue dans le cadre de cet essai 8, atteinte à 4 heures, est de 0,63.

On peut toutefois supposer que des résultats meilleurs pourraient être obtenus dans l'essai 8 en chauffant à une température plus élevée une composition contenant un peu plus d'anti-bactériens.

L'activité anti-bactérienne de la composition comprenant 0,2% en poids de 2-phénylphénol et 1% en poids d'essence de thym rouge est, après 4 heures, nettement plus élevée avec un mode de diffusion par combustion catalytique que dans celui mis en oeuvre dans l'essai 8, d'autant plus élevée que le temps de diffusion dans l'essai 5 a été de près de 5 fois moindre que dans l'essai 8, pour un poids de solvant vaporisé légèrement inférieur, de 12 g dans l'essai 5 et de 13,1 g dans l'essai 8.

Ceci démontre l'existence d'une troisième synergie associant cette composition anti-bactérienne au mode de diffusion particulièrement fine procurée par la vaporisation à l'aide du flacon à combustion catalytique

L'ensemble des données relevées lors des essais 1 à 8 a été regroupé dans le tableau ci-dessous, dans lequel les abréviations "ess.", "2-PP", "synth." et "*" signifient respectivement "essence de thym rouge", "2-phénylphénol", "thymol de synthèse", et "non mesuré":

| essai | Composition anti-bactérienne (% en poids) | ED à 23h | ED max. | ED nulle | ED à 168h |
|---|---|---|---|---|---|
| 1 | solvant seul | 0 | 0,41 | à 5h | 0 |
| 2 | 1% (ess.) | 0,52 | 0,52 | à 55h | 0 |
| 3 | 0,2% (2-PP) | 0 | 0,68 | à 8h | 0 |
| 4 | 1% (2-PP) | 0 | 0,70 | à 8h | 0 |
| 5 | 0,2% (2-PP) - 1% (ess.) | 1,01 | 1,01 | >168h | 0,4 |
| 6 | 0,2% (2-PP) - 1% (synth.) | 0,95 | 1,02 | >70h | * |
| 7 | 0,2% (2-PP) - 0,5% (synth.) | 0,82 | 0,9 | >70h | * |
| 8 | 0,2% (2-PP) - 1% (ess.) | 0 | 0,63 | à 55h | 0 |

La comparaison des valeurs d'ED des essais 5 et 8 met clairement en évidence que la diffusion à l'aide d'un système à combustion catalytique d'une composition anti-bactérienne selon l'invention permet d'obtenir une destruction de plus de 90% des bactéries présentes dans l'air, taux qui n'est jamais atteint avec le mode de diffusion retenu dans l'essai 8.

De façon surprenante, cette excellente activité anti-bactérienne présentée par le mode de diffusion par combustion catalytique associé à la composition anti-bactérienne selon la présente invention présente un effet rémanent extrêmement intéressant.

Cet effet rémanent est particulièrement remarquable dans le cas de la composition anti-bactérienne utilisée dans l'essai 5 puisque, 7 jours après l'initialisation de la combustion catalytique qui ne fonctionne par ailleurs que pendant une durée très courte de 25 minutes, l'ED est encore d'environ 0,4.

On constate donc que la diffusion, à l'aide d'un système à combustion catalytique, de compositions associant notamment du 2-phénylphénol et du thymol dans de très faibles proportions pondérales, assure une diffusion fine et optimale des dites compositions, permettant non seulement une réduction de la population bactérienne dans les heures qui suivent la diffusion de ces compositions, mais également une rémanence dans le temps.

Ce constat est corroboré par une analyse complémentaire conduite sur des bactéries pathogènes : le *Staphylococcus aureus* et le *Pseudomonas aeruginosa,* qui sont deux des cinq micro-organismes retenus par les normes AFNOR pour mesurer et valider l'activité anti-bactérienne par contact d'un composé.

Trois plaques, sur lesquelles a été ensemencé un nombre déterminé de *Pseudomonas aeruginosa,* ont été disposées respectivement à proximité, à 50 cm et à 1 m d'un flacon du type de celui représenté à la figure 1, comprenant la composition de l'essai 5 et muni d'un brûleur à combustion catalytique tel que décrit à la figure 2.

Après 30 minutes de diffusion de la composition de l'essai 5, il a été constaté une réduction importante, en l'espèce de 89%, du nombre de *Pseudomonas aeruginosa* sur la plaque située à proximité du flacon ; cette réduction diminue ensuite : elle est de 45% pour la plaque située à 50 cm et plus que de 7% pour celle située à 1 m.

Des résultats comparables ont été obtenus avec l'essai conduit sur le *Staphylococcus aureus,* selon le même protocole que celui décrit ci-dessus pour le *Pseudomonas aerugino*.

La réduction du nombre de *Staphylococcus aureus* est de 94% pour la plaque située à proximité du flacon, de 46% pour celle située à 50 cm et de 10% pour celle située à 1 m.

Ces deux dernières expériences démontrent clairement que, tout en ne mettant en oeuvre que de très faibles quantités de 2-phénylphénol et d'essence de thym rouge, une activité anti-bactérienne significative peut être obtenue, même en l'absence de tout contact direct avec la composition précitée, sur deux micro-organismes pathogènes, connus par ailleurs comme ayant une grande résistance aux agressions chimiques et physiques, nettement supérieure à la résistance des germes banaux de l'air. On peut en déduire que les mêmes germes présents dans l'air de cette enceinte seraient aussi détruits.

Ceci démontre que l'activité anti-bactérienne de la composition et du procédé selon la présente invention sur les germes contenus dans l'air d'une enceinte à traiter est intense non seulement contre les germes banaux de l'air mais également contre des germes pathogènes résistants pouvant être présents dans cet air.

Une utilisation de l'invention est donc envisageable pour lutter contre les germes banaux de l'air tels que *Rhodotorula rubra, Aspergillus niger, Penicillium,* et des bactéries pathogènes parmi lesquelles *Staphylococcus aureus* et *Pseudomonas aeruginosa.*

L'invention présente donc l'avantage double de n'utiliser que peu de composés actifs et donc de diffuser une composition anti-bactérienne ne présentant aucun danger pour la santé de l'utilisateur, par un troisième effet de synergie surprenant entre la composition anti-bactérienne et le procédé de diffusion de celle-ci.

Cet effet de synergie paraît encore plus marqué quand on utilise de l'essence de thym rouge.

L'économie réalisée grâce aux faibles quantités de composés actifs employées est encore augmentée par une mise en oeuvre relativement rapide du procédé.

En particulier, la durée de diffusion de la composition selon l'invention peut être augmentée, et ainsi permettre d'atteindre l'efficacité destructrice ED maximale plus rapidement.

Par ailleurs, il y a tout lieu de penser que la composition anti-bactérienne et le procédé selon la présente invention ont également un effet anti-microbien significatif, en particulier en matière de lutte contre les champignons, y compris leurs spores, les virus ainsi que les spores bactériennes.

## Revendications

1. Composition destinée à être diffusée dans l'air d'une enceinte à traiter pour lutter contre les bactéries contenues dans l'air de cette enceinte, **caractérisée en ce que** cette composition comprend du 2-phénylphénol, du thymol, naturel et/ou de synthèse, et un solvant adapté à être diffusé par vaporisation à chaud, le 2-phénylphénol et le thymol étant dissous dans ledit solvant.

2. Composition selon la revendication 1, **caractérisée en ce que** la proportion pondérale de 2-phénylphénol est comprise entre 0,05 et 1% et la proportion pondérale de thymol est comprise entre 0,05 et 5%.

3. Composition selon la revendication 2, **caractérisée en ce que** la proportion pondérale de 2-phénylphénol est comprise entre 0,1 et 0,3%, de préférence égale à 0,2%, et la proportion pondérale de thymol est comprise entre 0,2 et 2%, de préférence entre 0,5 et 1%.

4. Composition selon l'une quelconque des revendications 1 à 3, **caractérisée en ce qu'**elle comprend une proportion pondérale totale de 2-phénylphénol et de thymol comprise entre 0,1 et 5%, de préférence entre 0,2 et 1,2%.

5. Composition selon l'une des revendications 1 à 4, **caractérisée en ce qu'**elle comprend de l'essence de thym rouge.

6. Composition selon l'une des revendications 1 à 5, **caractérisée en ce qu'**elle comprend en outre un ou plusieurs autres composés, naturels ou de synthèse, par exemple, une matière parfumée.

7. Procédé de lutte contre les bactéries contenues dans l'air d'une enceinte à traiter comprenant une étape de diffusion, dans l'air de cette enceinte, d'une composition anti-bactérienne, **caractérisé en ce que** l'on diffuse la composition selon l'une des revendications 1 à 6 par vaporisation à chaud.

8. Procédé selon la revendication 7, **caractérisé en ce que** la vaporisation à chaud s'effectue par simple chauffage de la composition.

9. Procédé selon la revendication 7, **caractérisé en ce que** l'on diffuse ladite composition au moyen d'un brûleur à combustion catalytique, le solvant étant combustible.

10. Utilisation, pour la mise en oeuvre du procédé de lutte contre les bactéries selon la revendication 7 ou 9, d'un flacon (2) à combustion catalytique, adapté à contenir une composition combustible (4) et à recevoir, sur une embase (34) fixée à son goulot (10), un brûleur (6) à combustion catalytique, ledit brûleur (6) comportant une cavité (12) axiale ouverte vers le bas et recevant une mèche (8) trempant dans ladite composition (4).

11. Utilisation selon la revendication 10, **caractérisée en ce que** le brûleur (6) présente :
- une gorge annulaire (22) axiale s'étendant depuis la surface supérieure (24) du brûleur (6) et séparant la zone périphérique (14) annulaire supportant le catalyseur de la zone centrale (16) sans catalyseur formant zone de vaporisation, et/ou
- un canal (18) pour mettre à l'atmosphère la partie supérieure (20) de la cavité (12) recevant la mèche (8).

12. Utilisation, selon la revendication 10 ou 11, pour lutter contre le *Rhodotorula rubra*, l'*Aspergillus niger,* le *Penicillium*, le *Staphylococcus aureus* et le *Pseudomonas aeruginosa*.

## Patentansprüche

1. Zusammensetzung zur Verbreitung in der Luft eines zu behandelnden Raumes, um in der Luft dieses Raumes enthaltene Bakterien zu bekämpfen,
**dadurch gekennzeichnet,**
**dass** diese Zusammensetzung umfasst natürliches und/oder synthetisches 2-Phenylphenol und Thymol sowie ein Lösungsmittel, das so angepasst ist, dass dieses durch Wärmeverdampfung verbreitet werden kann, wobei das 2-Phenylphenol und das Thymol in dem Lösungsmittel gelöst sind.

2. Zusammensetzung nach Anspruch 1,
**dadurch gekennzeichnet,**
**dass** der Gewichtsanteil von 2-Phenylphenol zwischen 0,05 und 1% beträgt und der Gewichtsanteil des Thymols zwischen 0,05 und 5% beträgt.

3. Zusammensetzung nach Anspruch 2,
**dadurch gekennzeichnet,**
**dass** der Gewichtsanteil von 2-Phenylphenol zwischen 0,1 und 0.3%, vorzugsweise gleich 0,2% beträgt und der Gewichtsanteil von Thymol zwischen 0,2 und 2%, vorzugsweise zwischen 0,5 und 1% beträgt.

4. Zusammensetzung nach einem der Ansprüche 1 bis 3,
**dadurch gekennzeichnet,**
**dass** diese einen Gesamtgewichtsanteil von 2-Phenylphenol und Thymol zwischen 0,1 und 5%, vorzugsweise zwischen 0,2 und 1,2% aufweist.

5. Zusammensetzung nach einem der Ansprüche 1 bis 4,
**dadurch gekennzeichnet,**
**dass** sie eine Essenz von rotem Thymian aufweist.

6. Zusammensetzung nach einem der Ansprüche 1 bis 5,
**dadurch gekennzeichnet,**
**dass** sie ferner ein oder mehrere natürliche oder synthetische weitere Bestandteile aufweist, zum Beispiel einen Parfümstoff.

7. Verfahren zum Bekämpfen von Bakterien, die in der Luft eines zu behandelnden Raumes enthalten sind, mit einem Schritt der Verbreitung einer antibakteriellen Zusammensetzung in der Luft dieses Raumes,
**dadurch gekennzeichnet,**
**dass** die Zusammensetzung gemäß einem der Ansprüche 1 bis 6 durch Wärmeverdampfung verbreitet wird.

8. Verfahren nach Anspruch 7,
**dadurch gekennzeichnet,**
**dass** die Wärmeverdampfung durch eine einfache Erhitzung der Zusammensetzung ausgeführt wird.

9. Verfahren nach Anspruch 7,
**dadurch gekennzeichnet,**
**dass** die Zusammensetzung mit Hilfe eines Brenners zur katalytischen Verbrennung verbreitet wird, wobei das Lösungsmittel brennbar ist.

10. Verwendung eines Glaskolbens (2) zur katalytischen Verbrennung für die Durchführung des Verfahrens zur Bekämpfung von Bakterien gemäß Anspruch 7 oder 9, der so angepasst ist, dass dieser eine brennbare Zusammensetzung (4) enthalten kann und auf einem an seinem Hals (10) befestigten Ansatz (34) einen Brenner (6) zur katalytischen Verbrennung aufnehmen kann, wobei der Brenner (6) einen axialen Hohlraum (12) aufweist, der zur Unterseite offen ist und einen Docht (8) aufnimmt, der in die Zusammensetzung (4) eintaucht.

11. Verwendung nach Anspruch 10,
**dadurch gekennzeichnet,**
**dass** der Brenner (6) aufweist:
- einen axialen Rundhals (22), der sich bis zur oberen Oberfläche (24) des Brenners (6) erstreckt und die ringförmige Umfangszone (14) abtrennt, die den Katalysator der die Verdampfungszone bildenden, katalysatorfreien zentralen Zone (16) trägt und/oder
- einen Kanal (18), um den oberen Teil (20) des den Docht (8) aufnehmenden Hohlraums (12) mit Umgebungsluft zu versetzen.

12. Verwendung nach Anspruch 10 oder 11,
um r*hodotorula rubra, aspergillus niger, penicillium, staphylococcus aureus und* psoudomonas *aeruginosa* zu bekämpfen.

## Claims

1. Composition intended to be diffused in the air of a chamber to be treated in order to control the bacteria contained in the air of this chamber, **characterized in that** this composition comprises 2-phenylphenol, natural and/or synthetic thymol, and a solvent suitable for being diffused by hot spraying, the 2-phenylphenol and the thymol being dissolved in said solvent.

2. Composition according to Claim 1, **characterized in that** the weight proportion of 2-phenylphenol is between 0.05 and 1% and the weight proportion of thymol is between 0.05 and 5%.

3. Composition according to Claim 2, **characterized in that** the weight proportion of 2-phenylphenol is between 0.1 and 0.3%, preferably equal to 0.2%, and the weight proportion of thymol is between 0.2 and 2%, preferably between 0.5 and 1%.

4. Composition according to any one of Claims 1 to 3, **characterized in that** it comprises a total weight proportion of 2-phenylphenol and of thymol of between 0.1 and 5%, preferably between 0.2 and 1.2%.

5. Composition according to one of Claims 1 to 4, **characterized in that** it comprises essence of red thyme.

6. Composition according to one of Claims 1 to 5, **characterized in that** it also comprises one or more other natural or synthetic compounds, for example a fragranced material.

7. Method for controlling the bacteria contained in the air of a chamber to be treated, comprising a step consisting in diffusing an antibacterial composition in the air of this chamber, **characterized in that** the composition according to one of Claims 1 to 6 is diffused by hot spraying.

8. Method according to Claim 7, **characterized in that** the hot spraying is carried out by simple heating of the composition.

9. Method according to Claim 7, **characterized in that** said composition is diffused by means of a catalytic combustion burner, the solvent being combustible.

10. Use, for implementing the method for combating the bacteria according to Claim 7 or 9, of a catalytic combustion flask (2) suitable for containing a combustible composition (4) and for receiving, on a seating (34) attached to its neck (10), a catalytic combustion burner (6), said burner (6) comprising an axial cavity (12) opening downwards and receiving a wick (8) soaking in said composition (4).

11. Use according to Claim 10, **characterized in that** the burner (6) has:
- an axial annular groove (22) extending from the upper surface (24) of the burner (6) and separating the annular peripheral zone (14) carrying the catalyst from the central zone (16) without catalyst forming the spray zone, and/or
- a channel (18) for bringing into contact with the atmosphere the upper part (20) of the cavity (12) receiving the wick (8).

12. Use according to Claim 10 or 11, for controlling *Rhodotorula rubra, Aspergillus niger, Penicillium, Staphylococcus aureus* and *Pseudomonas aeruginosa*.
